# EUROPÄISCHE PATENTSCHRIFT

(11) **EP 0 546 548 B1**
(45) Veröffentlichungstag und Bekanntmachung des Hinweises auf die Patenterteilung: **02.05.1997**
(21) Anmeldenummer: 92121077.9
(22) Anmeldetag: 10.12.1992
(51) Int. Cl.: C07F 9/38, A61K 31/66, C07F 9/40, C07F 9/6506

(54) **Guanidinalkyl-1,1-bisphosphonsäurederivate, Verfahren zu ihrer Herstellung und ihre Verwendung**
Guanidinyl alkyl-1,1-bis phosphonic acid derivatives, process for their preparation and their use
Dérivés d'acides guanidinyl alkyl bis-1,1-phosphoniques, procédé pour les préparer et leur utilisation

(30) Priorität: 12.12.1991 DE 4140908; 09.04.1992 DE 4211976
(43) Veröffentlichungstag der Anmeldung: 16.06.1993
(73) Patentinhaber: HOECHST AKTIENGESELLSCHAFT, 65926 Frankfurt am Main (DE)
(72) Erfinder: Lang, Hans-Jochen, Dr., W-6238 Hofheim/Ts. (DE); Naumann, Christoph, Dr., W-6272 Niedernhausen (DE); Carniato, Denis, F-92140 Clamart (FR); Moura, Anne-Marie, F-75014 Paris (FR); Satoh, Ryochi, Tsurugashima-shi, Saitama (JP); Katoh, Masakazu, Hatoyama-machi, Saitama (JP)

(56) Entgegenhaltungen:
- EP-A- 186 405
- EP-A- 0 243 173
- EP-A- 0 282 309
- EP-A- 0 354 806
- PHOSPHORUS AND SULFUR, Band 11, nr. 3, 1981, US, Seite 311 - 322 LUDWIG MAIER
- ' Organische Phosphorverbindungen 75 '
- Ernst Juckner ; "Fortschritte der Arzneimittelforschung" Vol. 7, Seiten 306/307 (1964)
- Houben-Weyl, Band E4 (1983); Seite 609

## Beschreibung

Osteoporose ist eine häufig vorkommende Knochenerkrankung. Bei den verschiedenen Formen der Osteoporose kommt es zu einem starken Verlust an Knochengewebe, so daß schließlich die mechanische Stabilität des Knochens verloren geht. In gesunden Menschen ist die Rate, mit der Osteoclasten und Osteoplasten gebildet werden, so ausgebildet, daß Knochenbildung und Knochenresorption im Gleichgewicht stehen. Bei Osteoporose ist das Gleichgewicht gestört, so daß es zu einem Knochenabbau kommt.

L. Maier (Phosphorus and Sulfur, 1981, 11, Seiten 311-322) beschreibt bei der Herstellung von Aminomethylendiphosphinaten als Zwischenverbindung 1-((Aminoiminomethyl)amino)-methan-1,1-bisphosphonsäuretetraethylester. Es wird keine Verwendung für dieses Zwischenprodukt angegeben.

In dem Bestreben, wirksame Verbindungen zur Behandlung und Prophylaxe von Osteoporose mit geringen Nebenwirkungen zu erhalten, wurde nun überraschenderweise gefunden, daß die erfindungsgemäßen Guanidinalkyl-1,1-bisphosphonsäuren die Knochenresorption vermindern.

Die Erfindung betrifft die Verbindungen 2-((O,O,-Diisobutylphosphoryl)-(aminoiminomethyl)amino)-ethan-1,1-bisphosphonsäuretetraethylester. 2-((Aminoiminomethyl)amino)ethan-1,1-bisphosphonsäure, 4-((Bis(1,1-dimethylethoxycarbonyl)aminoiminomethyl)amino)butan-1,1 -bisphosphonsäure-tetraethylester, 2-((Benzimidazolaminoiminomethyl)amino)ethan-1,1-bisphosphonsäuretetraethylester, 2-((Benzimidazolaminoiminomethyl)amino)ethan-1,1-bisphosphonsäure und 4-((Aminoiminomethyl)amino)butan-1,1-bisphosphonsäure.

Die erfindungsgemäßen Verbindungen lassen sich dadurch herstellen; daß man
A) eine Verbindung der Formel la 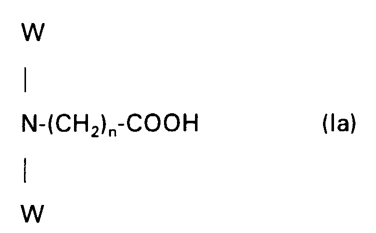 und/oder das entsprechende Anhydrid oder Säurechlorid der Verbindung der Formel Ia,
   wobei W eine Aminoschutzgruppe ist und n 2 oder 4 bedeutet,
   A1) mit einer Verbindung der Formel IIa und/oder IIb

      P(OR⁵)₃ (IIa)

      P(OR⁶)₃ (IIb)

      wobei R⁵ und R⁶ unabhängig voneinander (C₁-C₅)-Alkyl, geradkettig oder verzweigt, steht,
      zu einer Verbindung der Formel Ib 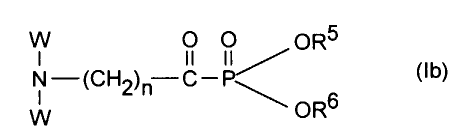 umsetzt, anschließend die Verbindung der Formel Ib
   A2) mit einer Verbindung der Formel IIc und/oder IId

      HP(O)(OR⁷)₂ (IIc)

      P(OH)(OR⁸)₂ (IId)

      in Gegenwart eines Dialkylamins oder Alkalialkanolats zu einer Verbindung der Formel Ic 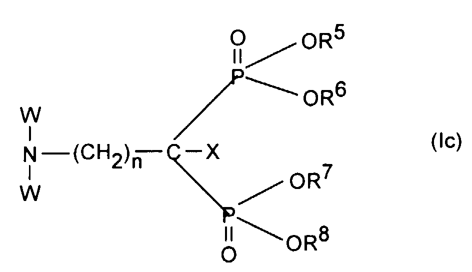 umsetzt, wobei R⁷ und R⁸ Wasserstoff oder (C₁-C₅)-Alkyl, geradkettig oder verzweigt, bedeutet und X für Wasserstoffatom oder Hydroxy steht,
      anschließend von der Verbindung der Formel Ic
   A3) die Aminoschutzgruppe abspaltet, und man die Verbindung der Formel Id erhält 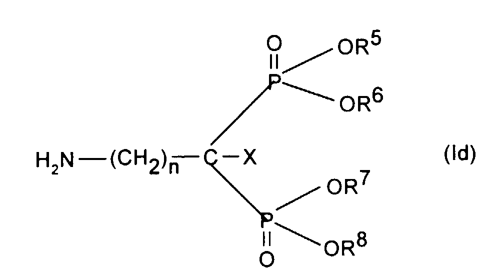
   A4) anschließend die Verbindung der Formel Id mit einer tautomeren Verbindung der Formel IIIa oder IIIb 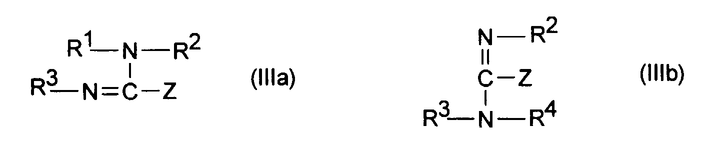 wobei Z ein Rest der nachstehenden Gruppe
      1) Methylmercaptan, 2) Ethylmercaptan oder 3) 3,5-Dimethylpyrazol-1-carbonsäureamidinnitrat ist
      und R¹, R², R³ oder R⁴ Wasserstoff, Benzimidazol oder O,O-Diisobutylphosphoryl bedeuten
      in Gegenwart eines inerten Lösungsmittels zu den tautomeren Verbindungen des Anspruchs 1 umsetzt, oder
B) eine Verbindung der Formel IV 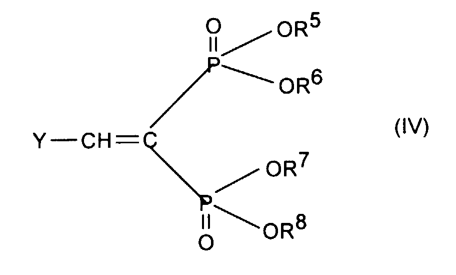 wobei R⁵, R⁶, R⁷, R⁸ die bei A1) bis A4) genannten Bedeutungen haben und Y Wasserstoff bedeutet,
   B1) mit einer Verbindung der Formel V, wobei Z Amino bedeutet, in Gegenwart eines inerten Lösungsmittels zu einem Bisphosphonsäureester des Anspruchs 1 umsetzt, und gegebenenfalls
   B2) den Bisphosphonsäureester des Anspruchs 1 in die entsprechende Bisphosphonsäure umsetzt, oder
C) eine Verbindung der Formel V 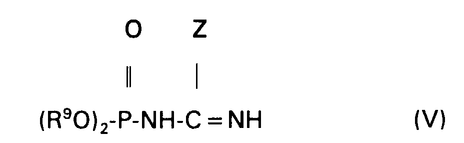 wobei Z die in A4) genannte Bedeutung hat und R⁹ (C₁-C₅)-Alkyl bedeutet,
   C1) mit einer Verbindung der Formel Id zu einem Bisphosphonsäureester des Anspruchs 1 umsetzt und anschließend gegebenenfalls
   C2) den Bisphosphonsäureester in die Bisphosphonsäure des Anspruchs 1 umsetzt, oder
D) eine Verbindung der Formel IV mit einer Verbindung der Formel IIIa oder Illb, wobei Z Amino bedeutet, und R¹, R², R³ und R⁴ die unter A4) genannten Bedeutungen haben, zu einer Verbindung des Anspruchs 1 umsetzt, oder
E) eine Verbindung der Formel Id
   E1) mit einer Verbindung der Formel VI 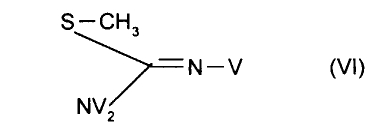 wobei V eine Aminoschutzgruppe ist umsetzt und man die Verbindung der Formel le erhält 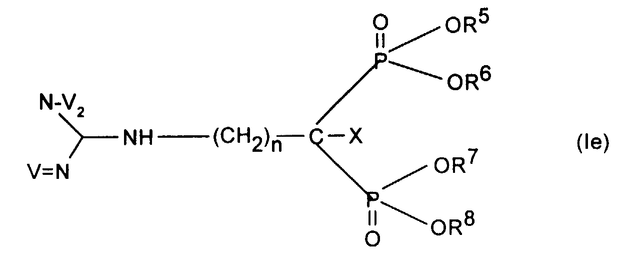
   E2) anschließend die gegebenenfalls vorhandene Aminoschutzgruppe abgespaltet, und man eine tautomere Verbindung des Anspruchs 1 erhält.
F) die in B2, C2 oder E2 erhaltenen Bisphosphonsäuren des Anspruchs 1 durch Umsetzung mit einer physiologisch verträglichen Base in die entsprechenden Salze überführt.

Bei dem Verfahrensschritt A1) geht man am besten so vor, daß man die Verbindung Ia oder das entsprechende Anhydrid oder Säurechlorid mit einem Phosphorigsäuretriester der Formel IIa oder IIb, gegebenenfalls in Anwesenheit einer Base, zu einer Verbindung der Formel Ib umsetzt.

Die Reaktionstemperaturen betragen von 0 °C bis 120 °C, bevorzugt von 0 °C bis 80 °C. Die Reaktionszeiten betragen etwa 1 bis 6 Stunden, bevorzugt 1 bis 3 Stunden. Die Beendigung der Reaktion kann beispielsweise mittels Dünnschichtchromatographie (DC) bestimmt werden.

Die für diesen Verfahrensschritt notwendigen Ausgangsstoffe der Formel Ia sind, sofern nicht käuflich, auf einfache Weise nach literaturbekannten Verfahren (J. H. Billman, W. F. Harting, Am. Soc. 70, S. 1473 (1948)) herstellbar. Geeignete Aminoschutzgruppen sind beispielsweise Phthaloyl- oder Benzoxycarbonyl-Reste (T. W. Greene, Protective Groups in Organic Synthesis).

Bei der Verfahrensvariante A2) geht man am besten so vor, daß man die Verbindung der Formel Ib) mit einem Phosphorigsäurediester der Formel IIc oder IId in Gegenwart eines Dialkylamins, beispielsweise Diethylamin, oder eines Alkalialkanolats, beispielsweise Natriummethylat, zur Verbindung der Formel Ic umsetzt.

Die Reaktionstemperaturen betragen von 0 °C bis 120 °C, bevorzugt von 0 °C bis 90 °C. Die Reaktionszeiten betragen etwa 1 bis 8 Stunden, bevorzugt 1 bis 4 Stunden. Die Beendigung der Reaktion kann beispielsweise mittels DC bestimmt werden.

Bei der Verfahrensvariante A3) spaltet man die Aminoschutzgruppe am besten wie folgt ab, indem man die alkoholische Lösung 1 bis 2 Stunden bis zum Siedepunkt erhitzt oder 1 bis 2 Tage bei Raumtemperatur inkubiert (J. C. Sheehan, Am. Soc. 74, S. 3822 (1952)).

Bei der Verfahrensvariante A4) geht man am besten so vor, daß man die Verbindung der Formel Id mit einer Verbindung der Formel IIIa oder IIIb in stöchiometrischen Verhältnis in einem inerten Lösungsmittel, z. B. Toluol, Dimethylformamid (DMF), Dimethylsulfoxid (DMSO), Pyridin, Tetrahydrofuran oder Dioxan, löst und unter Erwärmen umsetzt.

Die Reaktionstemperaturen betragen von 60 °C bis 180 °C, bevorzugt von 80 °C bis 120 °C. Die Reaktionszeiten betragen etwa 1 bis 22 Stunden, bevorzugt 1 bis 6 Stunden.

Die Beendigung der Reaktion kann durch DC bestimmt werden. Die Produkte der Reaktion lassen sich säulenchromatographisch reinigen, beispielsweise auf Kieselgel mit einer Mischung aus Essigester und Alkohol im Volumenverhältnis 5 : 1, oder allein mit Alkohol als Laufmittel.

Bei der Verfahrensvariante B) geht man am besten so vor, daß man die Verbindung IV oder das entsprechende Anhydrid oder Säurechlorid mit einem Phosphorigsäureester der Formel IIa oder IIb und einem Phosphorigsäurediester der Formel IIc oder IId in Gegenwart eines Dialkylamins, beispielsweise Diethylamin, oder eines Alkalialkanolats, beispielsweise Natriummethylat, zur Verbindung Ic umsetzt.

Die Reaktionstemperaturen betragen etwa 60 °C bis 180 °C, bevorzugt 80 °C bis 120 °C. Die Reaktionszeiten betragen etwa 1 bis 6 Stunden, bevorzugt 1 bis 3 Stunden.
Die Beendigung der Reaktion kann durch DC bestimmt werden. Die Produkte der Reaktion lassen sich säulenchromatographisch auf einer Kieselgelsäule reinigen, beispielsweise mit einem Laufmittel aus Essigester/Ethanol im Volumenverhältnis 5 : 1 oder mit Ethanol allein.
Die weitere Umsetzung erfolgt wie unter Verfahrensschritt A3) und A4) beschrieben.

Bei der Verfahrenvariante C) geht man am besten so vor, daß man eine Verbindung der Formel Ic mit einem Phosphorylierungsmittel wie beispielsweise Phosphortrioxid, Phosphortrihalogenid im Gemisch mit phosphoriger Säure oder Phosphorsäure, Phosphoroxychlorid oder Phosphorpentachlorid sowie Phosphortrichlorid und Chlor, zu einer Verbindung der Formel Ic umsetzt. Bevorzugt ist Phosphortrioxid, welches vorzugsweise durch Umsetzung von Phosphortrichlorid mit phosphoriger Säure oder durch Reaktion überschüssigen Phosphortrichlorids mit wasserhaltiger Phosphorsäure, z. B. mit handelsüblicher etwa 75%iger bis etwa 95%iger, vorzugsweise etwa 85%iger Phosphorsäure, in situ gebildet wird. Die Umsetzung wird vorteilhaft unter Erwärmen, z. B. auf etwa 70 °C bis etwa 120 °C, in einem geeigneten Lösungsmittel wie Tetrachlorethan, Trichlorethan, Chlorbenzol, Chlortoluol oder Paraffinöl oder ohne Lösungsmittel und anschließend unter hydrolytischer Aufarbeitung durchgeführt Die weitere Umsetzung erfolgt wie unter A3) und A4) beschrieben.

Bei der Verfahrensvariante D) geht man am besten so vor, daß man die Verbindung der Formel IIIa oder IIIb, wobei Z Amino bedeutet, in äquimolarer Menge oder in einem bis zu 3-fachen Überschuß, gegebenenfalls in einem inerten Lösungsmittel wie Dimethylsulfoxid (DMSO), Dimethylformamid (DMF), Toluol, (C₁-C₄)-Alkanol, Tetrahydrofuran (THF), Dioxan oder Diethylether mit einer Verbindung der Formel VIII unter Zusatz einer Base wie Kaliumcarbonat, Natriumcarbonat, Kaliumhydroxid, Triethylamin, Diethylamin oder wahlweise auch ohne Basenzusatz zu einer Verbindung des Anspruchs 1 umsetzt. Die Reaktionstemperaturen liegen bei etwa 25 °C bis 100 °C, vorzugsweise bei Verwendung eines Lösungsmittels bei 25 °C und dem Siedepunkt des Lösungsmittels, insbesondere bei 70 °C. Die Reaktionszeiten betragen 6 bis 48 Stunden, bevorzugt 12 bis 24 Stunden. Die Beendigung der Reaktion kann beispielsweise mittels Dünnschichtchromatographie bestimmt werden.
Zur Reinigung der Reaktionsmischung kann man die Reaktionsmischung auf einer Kieselgelsäule mit einem Laufmittelgemisch aus Essigester und Alkohol, Volumenverhätnis beispielsweise 6 : 1, reinigen. Verseifung der Phosphorsäureester der Verbindungen des Anspruchs 1 erfolgt z. B. durch Erhitzen unter Reflux in konzentrierter Salzsäure, oder durch Behandlung mit starken Säuren oder Trimethylsilylhalogenid in wasserfreien Lösungsmittel erhalten werden. Wasserfreie Bromwasserstoffsäure in Essigsäure kann direkt oder nach entsprechender Verdünnung verwendet werden, oder es wird Trimethylsilyliodid gelöst in einem Lösungsmittel wie Tetrachlorkohlenstoff, Dimethylformamid, Chloroform oder Toluol verwendet. Die Hydrolyse kann unter Kühlung oder Erhitzen ausgeführt werden, z. B. kann der Ester mit einem Trimethylsilylhalogenid unter Kühlung bei - 10 °C oder darunter umgesetzt werden, und man erhält ein teilweise verseiftes Produkt.

Die Ausgangsverbindungen der Verfahrensvariante D) sind für den Fall der Verbindung VIII auf einfache Weise nach literaturbekannten Verfahren herstellbar (EP-A-0 298 553).

Bei den Verfahrensvariante E) geht man am besten so vor, daß man eine Verbindung der Formel (Id) in 1N Salzsäure löst und eindampft. Der Rückstand wird dann in einem Alkohol aufgenommen und mit einer Verbindung der Formel (VI) versetzt. Das Gemisch wird dann zum Sieden erhitzt und zu einer Verbindung des Anspruchs 1 umgesetzt.

Bei der Verfahrensvariante F) geht man am besten so vor, daß man die Verbindungen des Anspruchs 1 unter Zusatz einer Base, z. B. Natronlauge oder Kalilauge, bei Raumtemperatur neutralisiert und durch Zugabe eines Lösungsmittels, z. B. Ethanol oder Hexan, ausfällt.

Die Erfindung betrifft auch Arzneimittel, die mindestens eine Verbindung des Anspruchs 1 enthält, neben pharmazeutisch geeigneten und physiologisch verträglichen Hilfs- und Trägerstoffen, Verdünnungsmitteln und/oder anderen Wirkstoffen.

Die Erfindung betrifft ferner die Verwendung der Verbindungen des Anspruchs 1 zur Prophylaxe und Behandlung von Osteoporose.

Die erfindungsgemäßen Arzneimittel können oberflächlich, percutan, nasal, intravenös, intramuskulär, intraperitoneal, subcutan, intraartikulär, periartikulär, rektal oder oral verabreicht werden.

Die Herstellung der erfindungsgemäßen Arzneimittel zur Prophylaxe und Behandlung von Osteoporose erfolgt, indem mindestens eine Verbindung des Anspruchs 1 gegebenenfalls mit Hilfs- und/oder Trägerstoffen in eine geeignete Darreichungsform gebracht wird. Die Hilfs- und Trägerstoffe stammen aus der Gruppe der Trägermittel, Konservierungsstoffe und anderer üblicher Hilfsstoffe.

Zum Beispiel können für orale Darreichungsformen Hilfsstoffe wie Stärken, z. B. Kartoffel-, Mais- oder Weizenstärke, Cellulose bzw. deren Derivate, insbesondere mikrokristalline Cellulose, Siliziumdioxid, verschiedene Zucker wie Milchzucker, Magnesiumcarbonat und/oder Calciumphosphate verwendet werden. Weiterhin ist es vorteilhaft, den oralen Darreichungsformen Hilfsstoffe zuzusetzen, die die Verträglichkeit der Medikamente verbessern, wie z. B. Schleimbildner und Harze. Zwecks besserer Verträglichkeit können die Medikamente auch in Form von magensaftunlöslichen Kapseln verabreicht werden. Darüber hinaus kann es vorteilhaft sein, der Darreichungsform, bzw. einer Komponente eines Kombinationspräparates, ein Retardierungsmittel zuzusetzen, gegebenenfalls in Form von permeablen Membranen, wie z. B. solche auf Cellulose- oder Polystrolharzbasis oder Ionenaustauscher.

Die anzuwendende Dosierung der erfindungsgemäßen Arzneimittel ist abhängig von verschiedenen Faktoren wie Darreichungsform des Medikamentes und Zustand, Gewicht und Schwere der Erkrankung des Patienten. Eine Tagesdosis von ca. 5000 mg der erfindungsgemäßen Arzneimittel sollte jedoch nur kurzzeitig überschritten werden. Bevorzugt sind etwa 10 bis 2500 mg als Tagesdosis für einen Menschen von 70 kg Körpergewicht. Die Verabreichung der Tagesdosis der erfindungsgemäßen Arzneimittel kann in Form einer einzelnen Verabreichung oder in mehreren kleinen Dosen erfolgen. Bevorzugt ist die Verabreichung in 3 bis 8 Dosen pro Tag.

Nachfolgend ist die Erfindung an Hand von Beispielen näher erläutert. Die Prozentangaben beziehen sich auf Volumenprozente, falls nicht anders angegeben.

### Referenz-Beispiel 1

### Herstellung von Diethylphosphitguanidin

9,6 g (100 mMol) Guanidinhydrochlorid werden in 8 g (200 mMol) Natriumhydroxid, welche in 50 ml Wasser gelöst ist, und 20 ml Ethanol aufgenommen und auf 0 °C gekühlt. Hierzu gibt man eine Lösung aus 13,8 g (100 mMol) Diethylphosphit in 50 ml Tetrachlorkohlenstoff und rührt 24 Stunden bei 25 °C. Anschließend gibt man 30 ml Wasser hinzu und extrahiert mit Methylenchlorid (3 X 30 ml). Die organischen Phasen werden vereint und getrocknet (K₂CO₃). Nach dem Einrotieren erhält man ein Öl, welches teilweise kristallisiert. Das Rohprodukt wird aus 100 ml Essigester umkristalliert. Man erhält nach Absaugen ein farbloses Produkt.
- Ausbeute:: 5,7 g (24 % d. Th.)
- Schmelzpunkt:: 112 - 113 °C

| | | | | |
|---|---|---|---|---|
| Analyse: | berechnet | C = 30.6 | H = 7.2 | N = 21.4 |
| | gefunden | C = 30.2 | H = 6.9 | N = 21.7 |

³¹P-NMR-Spektroskopie:
(D₂O) δP = 8.54 ppm.

### Beispiel 1

### Herstellung von Diisobutylphosphitguanidin

9,6 g (100 mMol) Guanidinhydrochlorid werden in 8,0 g (200 mMol) Natriumhydroxid, welche in 50 ml Wasser gelöst ist und 20 ml Ethanol aufgenommen und auf 0 °C gekühlt. Hierzu tropft man innerhalb von 15 min eine Lösung von 16,2 g (834 mMol) Diisobutylphosphit in 50 ml Tetrachlorkohlenstoff zu. Anschließend wird die Kühlung entfernt und man rührt 4 Stunden bei 25 °C nach. Die organische Phase wird abgetrennt und die erhaltene wässrige Phase noch 2 mal mit je 50 ml Chloroform extrahiert. Die vereinigten Extrakte werden mit Kaliumcarbonat getrocknet. Nach Einrotieren und Trocknen erhält man 18,3 g Rohprodukt, welches aus 180 ml Ethylacetat umkristallisiert wird.
- Ausbeute:: 13,3 g (64,7 %)
- Schmelzpunkt:: 152 - 156 °C

| | | | | |
|---|---|---|---|---|
| Analyse: | berechnet | C = 43.0 | H = 8.83 | N = 16.7 |
| | gefunden | C = 42.8 | H = 8.70 | N = 16.5 |

¹H-NMR-Spektroskopie:
- (CDCl₃): 0.93 (d, 7.5 Hz, 12 H, aliphat); 1.90 (mc, 2H, aliphat); 3.65 (t, 7.5 Hz, 4 H, aliphat).
³¹P NMR-Spektroskopie:
- (CDCl₃): δP = 7.87 ppm.

### Beispiel 2

### Herstellung von 2-((O,O-Diisobutylphosphoryl)-(aminoiminomethyl)amino)-ethan-1,1-bisphosphonsäuretetraethylester

10 g (40 mMol) Diisobutylphosphitguanidin und 12,4 g (41mMol) Vinyldiphosphonsäuretetraethylester werden in 150 ml absolutem Tetrahydrofuran gelöst. Hierzu gibt man 0,5 g Kaliumcarbonat und erhitzt 8 Stunden zum sieden. Nach Abfiltrieren und Einrotieren erhält man 24 g Rohsubstanz als Öl. Die Substanz wird über eine Kieselgelsäule chromatographiert. Als Laufmittel dient Essigester mit 20 % Ethanol.
- Ausbeute:: 11,4 g (51 % d. Th.)

| | | | | |
|---|---|---|---|---|
| Analyse: | berechnet | C = 41.3 | H = 8.0 | N = 7.6 |
| | gefunden | C = 41.1 | H = 8.0 | N = 7.4 |

¹H-NMR-Spektroskopie:
- (CDCl₃): 0.95 (d, 7.5 Hz, 12H, aliphat); 1.34 (mc, 12H, aliphat); 1.92 (mc, 2H, aliphat); 3.45 (mc, 1H); 3.82 (td, 2H); 3,74 (mc, 4H); 4,10-4.28 (m, 8H, aliphat).
³¹P-NMR-Spektroskopie:
- (CDCl₃): δP₁ = 9.11, δP₂ = 21.94 ppm.

### Referenz-Beispiel 2

### Herstellung von 2-Phthaloyl-ethan-1-monoethylphosphonat-1-bisethylphosphonat

30 g (146 mMol) Phthaloylglycin, 26,8 g (225 mMol) Thionylchlorid und 1 ml Dimethylformamid werden vorgelegt und unter heftigem Rühren für 4 Stunden auf 50 - 55 °C erwärmt. Nach Beendigung der Gasentwicklung wird das überschüssige Thionylchlorid unter verminderten Druck entfernt.
Zu dem erhaltenen Säurechlorid werden dann 49,9 g (300 mMol) Triethylphosphit, 41,4 g (296 mMol) Diethylphosphit und 2 g Triethylamin langsam zugetropft. Durch Zugabe der genannten Komponenten wird eine Reaktionstemperatur von 60 - 70 °C erreicht. Nach Zugabe wird die Reaktionstemperatur langsam auf 115 °C in der Weise gesteigert, daß die Gasentwicklung nicht abbricht. Nach Erreichen der 115 °C wird diese Temperatur eine weitere Stunde gehalten und dann die Leichtsieder bei 80 °C im Hochvakuum (etwa 10⁻³ Torr) abgezogen. Das Rohprodukt wird über eine Kieselgelsäule mit Essigester als Laufmittel gereinigt. Dabei wird die Reinsubstanz als hellgelbes Öl erhalten.
- Ausbeute:: 28,4 g (46,1 % d. Th.)

| | | | | |
|---|---|---|---|---|
| Analyse: | gefunden | C = 45.8 | H = 5.5 | N = 3.3 |
| | berechnet | C = 46.1 | H = 5.5 | N = 3.7 |

³¹P-NMR-Spektroskopie:
- (CDCl₃): δP₁ = -0.58, δP₂ = 16.63 ppm.

### Referenz-Beispiel 3

### Herstellung von 2-Amino-ethan-1-monoethylphosphonat-1-bisethylphosphonat

8 g (19,1 mMol) 2-Phthaloyl-ethan-1-monoethylphosphonat-1-bisethylphosphonat wird in 50 ml Ethanol gelöst und mit 1,34 g (21,4 mMol) Hydrazinhydrat versetzt und eine Stunde zum Sieden erhitzt. Nach Abziehen des Lösungsmittels bleibt ein farbloser Rückstand zurück. Dieser wird bei 0 °C in 35 ml 5N Essigsäure verrieben und der entstehende Niederschlag abgetrennt. Das Filtrat wird bei 0 °C mit 2N Natronlauge auf PH 7 gestellt und 3 mal mit 60 ml Methylenchlorid extrahiert. Die wässrige Phase wird auf PH 9 gestellt und erneut 2 mal mit 50 ml Methylenchlorid extrahiert. Die Extrakte werden vereinigt und 3 mal mit 80 ml einer zehnprozentigen Natriumchloridlösung gewaschen. Anschließend wird die organische Phase über Magnesiumsulfat getrocknet, filtriert und einrotiert. Das Rohprodukt wird über eine Kieselgelsäule mit Ethanol als Laufmittel gereinigt. Dabei wird das Produkt als hellgelbes Öl erhalten.
- Ausbeute:: 3,4 g (61,8 % d. Th.)

| | | | | |
|---|---|---|---|---|
| Analyse: | berechnet | C = 33.3 | H = 7.3 | N = 4.8 |
| | gefunden | C =33.6 | H = 7.1 | N = 4.6 |

³¹P-NMR-Spektroskopie:
- (CDCl₃): δP₁ = -0.39, δP₂ = 18.85 ppm.

### Beispiel 3

### Herstellung von 2-((Aminoiminomethyl)amino)-ethan-1,1-bis-phosphonsäure

1 g (1,8 mMol) 2-((O,O-Diisobutylphosphoryl)-(aminoiminomethyl)amino)-ethan-1,1-bisphosphonsäuretetraethylester werden in 20 ml konzentrierter Salzsäure 5 Stunden zum Sieden erhitzt. Gleichzeitig wird Ethanol, der sich bildet abdestilliert. Anschließend wird überschüssige Salzsäure abgezogen. Das zurückbleibende Öl wird in 30 ml Aceton mit 10 % Methanol verrührt und abdekantiert. Anschließend wird mit 20 ml Methanol/10% Wasser aufgekocht und 24 Stunden zur Kristallisation stehengelassen. Die farblosen Kristalle werden abgesaugt und getrocknet.
- Ausbeute:: 0,35 g (79,5 % d. Th.)
- Schmelzpunkt:: 230 °C (u. Zers.)

| | | | | |
|---|---|---|---|---|
| Analyse: | berechnet | C = 14.6 | H = 4.5 | N = 17.0 |
| | gefunden | C = 14.3 | H = 4.8 | N = 17.4 |

¹H-NMR-Spektroskopie:
- (D₂O):: 1.92 (tt, 1H); 3.54 (td. 2H).
³¹P-NMR-Spektroskopie :
- (NaOD): δP₁ = 18.88 ppm

### Beispiel 4

### Herstellung von 4-((Aminoiminomethyl)amino)butan-1,1-bis Phosphonsäure

Eine Lösung von 0,69 g (2 mMol) 4-Aminobutan-1,1-bis-Phosphonsäuretetraethylester (nach DE 3 225 469 A1, 05.01.1984) und 0,402 g (2 mmol) 3,5-Dimethylpyrazol-1-carboxamidin nitrat in 10 ml DMF wird 3 Stunden zum Sieden erhitzt. Dann wird das DMF im Vakuum abdestilliert und der Rückstand in wenig Wasser gelöst. Nach Extraktion mit Diethylether und Essigester (die organischen Extrakte werden verworfen) wird die wässrige Phase im Vakuum destilliert. Es bleibt ein dunkles Öl (0,89 g) zurück. Dieses wird in 15 ml konzentrierter Salzsäure 8 Stunden zum Sieden erhitzt. Die überschüssige Salzsäure wird abdestilliert und der Rückstand mit wenig Wasser, DMF und Aceton versetzt. Der Niederschlag wird abgesaugt und im Vakuum getrocknet.
- Ausbeute:: 137 mg
- Schmelzpunkt:: 190-195 °C
Massenspektroskopie:
m/z = M+-H⁺-= 274

### Referenz-Beispiel 4

### Herstellung von 2-(4-Trifluormethyl)-phenyl-ethen-1,1-bisphosphonsäuretetraethylester

50 ml absolutes Tetrahydrofuran werden auf - 10 °C abgekühlt und es werden langsam 7,42 g (39 mMol) Titantetrachlorid zugetropft. Hierzu gibt man 2,84 g (16,3 mMol) p-Trifluormethylbenzaldehyd und 5,63 g (19,6 mMol) Methandiphosphonsäuretetraethylester und läßt die Reaktionstemperatur auf 0 °C steigen. Danach tropft man 7,75 g (76,6 Mmol) Triethylamin in den Reaktionsansatz, wobei die Temperatur nicht über 5 °C steigt. Anschließend wird der Ansatz 3 Stunden bei Raumtemperatur gerühren und anschließend gibt man 100 ml Eis/Wasser hinzu. Der erhaltene Ansatz wird mit Diethylether (3 x 200 ml) extrahiert. Die vereinigten Etherextrakte werden solange mit Wasser gewaschen bis der Extrakt neutral ist. Anschließend wird die Etherphase mit einer gesättigten Kochsalzlösung gewaschen und über Natriumsulfat getrocknet. Das Rohprodukt wird über eine Kieselgelsäule mit Aceton gereinigt. Man erhält ein hellgelbes Öl.
- Ausbeute:: 2,5 g (34,7 % d. Th.)

| | | | |
|---|---|---|---|
| Analyse: | berechnet | C = 45.9 | H = 5.6 |
| | gefunden | C = 46.4 | H = 5.3 |

¹H-NMR-Spektroskopie:
- (CDCl₃): 1.14 - 1.43 (m, 12H); 3.94 - 4.32 (m, 8H); 7.64 - 7.84 (m, 4H, aromat); 8.18 - 8.45 (m, 1H, aliphat).

### Referenz-Beispiel 5

### Herstellung von N,N-Dibenzylaminomethan-1,1-bisphosphonsäuretetraethylester

197,3 g (1 Mol) Dibenzylamin und 171,9 g (1,16 Mol) Ameisensäureorthoethylester und 290 g (2,1 Mol) Diethylphosphit werden innerhalb von 3 Stunden unter gleichzeitigem Abdestillieren von Ethanol auf 150 °C erhitzt. Anschließend werden die Leichtsieder im Hochvakuum (etwa 10⁻³ Torr) entfernt und das Rohprodukt über eine Kieselgelsäule mit Essigester als Laufmittel gereinigt. Man erhält 197 g Rohprodukt. 60 g werden über eine Kieselgelsäule mit Essigester als Laufmittel gereingt, wobei man die Reinsubstanz als hellgelbes Öl erhält.
- Ausbeute:: 20,8 g (40,7 % d. Th.)

| | | | | |
|---|---|---|---|---|
| Analyse: | berechnet | C = 57.1 | H = 7.3 | N = 2.9 |
| | gefunden | C = 57.4 | H = 7.4 | N = 3.2 |

### Referenz-Beispiel 6

### Herstellung von 1-Aminomethan-1,1-bisphosphonsäuretetraethylester

17,1 g (35.4 mMol) N,N-Dibenzylaminomethan-1,1-bis-phosphonsäuretetraethylester werden in 170 ml Ethanol gelöst und 3,4 g 5 %ig Pd/C zugegeben. Die Hydrierung erfolgt bei Normaldruck. Nach Aufnahme von 100 ml Wasserstoff gibt man erneut 3,4 g 5 %ig Pd/C hinzu und vollendet die Hydrierung bei Normaldruck und Raumtemperatur (Reaktionsdauer ca. 16 Stunden). Anschließend wird der Katalysator abfiltriert und abgesaugt. Nach Abziehen des Lösungsmittels erhält man 10,9 g Rohprodukt. Die Reinigung erfolgt über eine Kieselgelsäule mit Ethanol als Laufmittel.
- Ausbeute:: 9,9 g (92,5 % d. Th.)

| | | | | |
|---|---|---|---|---|
| Analyse: | berechnet | C = 35.7 | H = 7.7 | N = 4.6 |
| | gefunden | C = 35.3 | H = 8.1 | N = 4.5 |

³¹P-NMR-Spektroskopie:
- (CDCl₃): δP = 20.48 ppm.

### Referenz-Beispiel 7

### Herstellung von O,O-Diisobutylphosphoryl S-ethyl isothiourea

18,5 g (0,1 Mol) S-Ethylisothioharnstoffhydrobromid werden in 50 ml Wasser, 20 ml Ethanol und 8 g (0,2 Mol) Natriumhydroxid gelöst und auf 0 °C abgekühlt. Eine Lösung von 19,4 g (0,1 Mol) Diisobutylphosphit in 50 ml Tetrachlorkohlenstoff werden innerhalb von 10 Min zugetropft. Nach Entfernen der Kühlung läßt man die Reaktion auf Raumtemperatur kommen und rührt für weitere 4 Stunden. Nach Abtrennen der organischen Phase wird die wässrige Phase nochmals 2 mal mit 100 ml Chloroform extrahiert über Kaliumcarbonat getrocknet, filtriert und einrotiert. Man erhält O,O-Diisobutylphosphoryl-S-ethyl-isothiourea als orangefarbenes Öl.
- Ausbeute:: 24,9 g (84,1 % d. Th.)

| | | | | |
|---|---|---|---|---|
| Analyse: | berechnet | C = 44.5 | H = 8.5 | N = 9.5 |
| | gefunden | C = 44.8 | H = 8.1 | N = 9.3 |

³¹P-NMR-Spektroskopie:
- (CDCl₃): δP = 4.29 ppm.

### Referenz-Beispiel 8

### Herstellung von 2-((Aminoiminomethyl)amino)-ethan-1,1-bis-phosphonsäure aus 2-Amino-ethan-1-monoethylphosphonat-1-bisethylphosphonat

4 g (13,8 mMol) 2-Amino-ethan-1-monoethylphosphonat-1-bisethylphosphonat und 4,15 g (14 mMol) O,O-Diisobutylphosphoryl S-ethyl isothiourea werden in 40 ml Dimethylformamid (DMF) gelöst und zum Sieden erhitzt. Nach 6 Stunden wird das DMF abgezogen und das erhaltene Rohgemisch wie in Beispiel 3 beschrieben aufgearbeitet und weiterverarbeitet.
- Ausbeute:: 1,8 g
³¹P-NMR-Spektroskopie:
- (NaOD): δP = 18.9 ppm.

### Beispiel 5

### Synthese von 4-((Bis(1,1-dimethylethoxycarbonyl)aminoiminomethyl)amino)butan-1,1-bisphosphonsäure-tetraethylester

Die Vorstufe 4-Aminobutan-1,1-bisphosphonsäuretetraethylester wird wie in DE 3 255 469 A1 beschrieben hergestellt. N,N'bis-(1,1-dimethylethoxycarbonyl)-S-methylisothiourea wird wie in der Literaturstelle K. Niwak, Roczniki, Chem. Ber. 43 (1), 23, 1969 beschrieben, hergestellt.
Eine Lösung von 1,8 g (6,2 mMol) N,N'-bis-(1,1-dimethylethoxycarbonyl)-S-methylisothiourea und 4,4 g (12,7 mMol) 4-Aminobutan-1,1-bisphosphonsäuretetraethylester werden in 50 ml Tetrahydrofuran (THF) 2 Tage auf 40 °C erwärmt. Anschließend wird die organische Phase mit einer gesättigten NaCl-Lösung gewaschen, über MgSO₄ getrocknet und das THF unter verminderten Druck entfernt. Das erhaltene Rohprodukt wird an Florisil (60 - 100 Mesh) chromatographiert. Das Laufmittel besteht aus Methylenchlorid/Methanol = 98/2. Hierbei werden 2,95 g (81 %) Reinsubstanz als farbloses Öl erhalten.
IR-Spektroskopie:
- (CHCl₃): *v* = 3300, 3285 (=C-NH); 1722, 1636 (C=O); 1617 (C=N) cm⁻¹
¹H-NMR-Spektroskopie:
- (CDCl₃): 1.34 (t, 12H, OCH₂CH₃); 1.49 (s, 18H, CH₃); 1.8 - 2.1 (m, 4H, N-CH₂CH₂CH₂CH); 2.33 (tt, P-CH-P), 3.45 (m, 2H, N-CH₂); 4.19 (m, 8H, OCH₂CH₃).

### Beispiel 6

### Synthese von 4-((Aminoiminomethyl)amino)butan-1,1-bisphosphonsäure

Eine Lösung von 0,92 g (2,56 mMol) 4-(Bis(1,1-dimethylethoxycarbonyl)aminoiminomethyl)amino)butan-1,1-bisphosphonsäuretetraethylester in 15 ml konzentrierter Salzsäure wird 12 Stunden zum Sieden erhitzt. Nach dem Einengen wird der Rückstand mit Ethanol versetzt. Der Niederschlag wird unter verminderten Druck abgesaugt und unter vermindertem Druck getrocknet. Die Umkristallisation erfolgt aus Ethanol/Wasser.
- Ausbeute:: 115 mg
- Schmelzpunkt:: 260 °C

| | | | | | |
|---|---|---|---|---|---|
| Analyse: | berechnet | C = 21.8 | H = 5.5 | N = 15.3 | P = 22.4 |
| | gefunden | C = 22.0 | H = 5.6 | N = 14.9 | P = 22.5 |

¹H-NMR-Spektroskopie:
- (D₂O/NaOD): 1.60 (t, 1H, P-CH-P); 1.79 (m, 4H, CH₂CH₂); 3.11 (m, 2H, N-CH₂).
Massenspektroskopie:
m/z = M + H ⁺ = 276.

### Beispiel 7

### Synthese von 2-((Benzimidazolaminoiminomethyl)amino)ethan-1,1-bisphosphonsäuretetraethylester

2,0 g (11,4 mMol) frisch umkristallisiertes Benzimidazolguanidin werden zusammen mit 3,4 g Ethyliden-1,1-bisphosphonsäuretetraethylester in 50 ml Ethanol 30 Minuten zum Sieden erhitzt. Anschließend wird das Ethanol im Vakuum entfernt. Durch Zugabe von Diethylether zu dem erhaltenen Öl erhält man das gewünschte Produkt in Form eines farblosen Pulvers.
- Ausbeute:: 4,9 g
- Schmelzpunkt:: 143°C

| | | | | |
|---|---|---|---|---|
| Analyse: | berechnet | C = 45,47 | H = 6,57 | N = 3,10 |
| | gefunden | C = 45,1 | H = 5,9 | N = 3,2 |

¹H-NMR-Spektroskopie:
(CDCl₃/TMS) 1,11-1,29 (m,12H,OCH₂CH₃); 3,75-3,93 (tt,1H,methin-H); 3,96-4,11 (m,8H,OCH₂CH₃); 4,50-4,61 (m,2H,-CH₂-); 7,00-7,11 (2H,aromaten-H); 7,18-7,29 (m, 1H,aromaten-H); 7,36-7,43 (m, 1H,aromaten-H).
³¹P-NMR-Spektroskopie:
(CDCl₃) 21,43 ppm

### Beispiel 8

### Synthese von 2-((Benzimidazolaminoiminomethyl)amino)ethan-1,1-bisphosphonsäure

1 g (2,1 mMol) der Substanz aus Beispiel 15 werden 4,5 Stunden mit 20 ml konzentrierter Salzsäure refluxiert.Nach Entfernen der überschüssigen Salzsäure im Vakuum wird das erhaltene zähe Öl aus Wasser umkristallisiert. Man erhält die Zielverbindung als farbloses Pulver.
- Ausbeute:: 250 mg
- Schmelzpunkt:: > 230°C

| | | | | |
|---|---|---|---|---|
| Analyse: | berechnet | C = 33,07 | H = 4,16 | N = 19.28 |
| | gefunden | C = 32,8 | H = 3,9 | N = 19,5 |

¹H-NMR-Spektroskopie:
(D₂O/NaOD): 2,63-2,83 (tt,1H,methin-H); 4,43-4,63 (m,2H,-CH₂-); 7,00-7,09 (2H,aromaten-H); 7,33-7,47 (1H,aromaten-H); 7,57-7,70 (1H,aromaten-H).
³¹P-NMR-Spektroskopie:
(D₂O/NaOD) 18,55 ppm.

Die Wirksamkeit der erfindungsgemäßen Verbindungen wird in vitro in folgenden Versuchen nachgewiesen.

Die Knochenresorption wird anhand der Analyse von ⁴⁵Ca-Freisetzung aus Schädeldecken von fötalen Ratten, die 20 Tage alt waren, bestimmt. Die Knochen werden markiert indem trächtigen Ratten 200 µCi/kg ⁴⁵CaCl₂ injiziert wird, 2 Tage bevor die Schädeldecke der Föten seziert wird.

### 1. Kultivierung der Knochen

Die Schädeldecke der Föten wird in zwei Hälften geteilt. Eine Schädeldeckenhälfte dient der Kontrolle, die andere Hälfte wird mit den erfindungsgemäßen Verbindungen inkubiert.
Jede Schädelhälfte wird in einer sterilen Plastikschale kultiviert. Das Kultivierungsmedium (BGJb-Medium, Gibco) enthält je ml 10 % fötales Kälberserum, Penicillin-Streptomycin (100 000 Units/l, Gibco) und Ascorbinsäure (50 mg/l). Die Schädelhälften werden bei 37 °C, in einer Gasatmosphäre von 5 % CO₂ und 95 % O₂ inkubiert. Nach 24 Stunden wird das Kultivierungsmedium durch frisches ersetzt, die erfindungsgemäßen Verbindungen und Parathyroid-Hormon (10⁻⁷ M) werden zugefügt und weitere 48 Stunden inkubiert. Der Kontrolle wird Parathyroid. Hormon (10⁻⁷ M Sigma) zugefügt.
Am Ende des Experiments wird die Aktivität von ⁴⁵Ca im Kulturmedium und im Knochen bestimmt.
Die Ergebnisse in Tabelle 1 zeigen die Inhibierung der ⁴⁵Ca-Freisetzung ins Kulturmedium in Prozent. Die Ergebnisse sind der Durchschnittswert von 3 bis 5 Experimenten.

**Tabelle 1:**

| Inhibierung der ⁴⁵Ca-Freisetzung | | | |
|---|---|---|---|
| Präparat Beispiel | Konzentration der Präparate | | |
| | 10⁻⁷ M | 10⁻⁶ M | 10⁻⁵ M |
| 6 | n. b. | 17,5% | 30,6 % |
| 14 | 31 % | 18 % | k. E. |
| n. b. = nicht bestimmt k. E. = kein Effekt | | | |

## Patentansprüche

1. 2-(O,O-Diisobutylphosphoryl)-(aminoiminomethyl)amino-ethan-1,1-bisphosphonsäuretetraethylester, 2-((Aminoiminomethyl)amino)ethan-1,1-bisphosphonsäure, 2-((Benzimidazolaminoiminomethyl)amino)-ethan-1,1-bisphosphonsäuretetraethylester, 2-(Benzimidazolaminoiminomethyl)aminoethan-1,1-bisphosphonsäure, 4-(Aminoiminomethyl)amino-butan-1,1-bisphosphonsäure.

2. Verfahren zur Herstellung einer Verbindung gemäß Anspruch 1, dadurch gekennzeichnet, daß man
A) eine Verbindung der Formel la 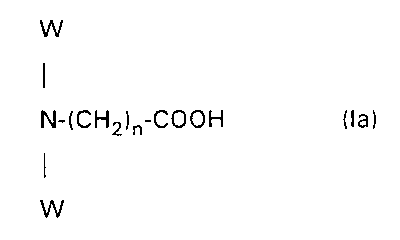 und/oder das entsprechende Anhydrid oder Säurechlorid der Verbindung der Formel la,
wobei W eine Aminoschutzgruppe ist und n 2 oder 4 bedeutet,
A1) mit einer Verbindung der Formel IIa und/oder IIb
P(OR⁵)₃ (IIa)
P(OR⁶)₃ (IIb)
wobei R⁵ und R⁶ unabhängig voneinander (C₁-C₅)-Alkyl, geradkettig oder verzweigt, steht,
zu einer Verbindung der Formel Ib 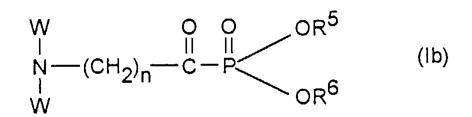 umsetzt, anschließend die Verbindung der Formel Ib
A2) mit einer Verbindung der Formel llc und/oder IId
HP(O)(OR⁷)₂ (IIc)
P(OH)(OR⁸)₂ (IId)
in Gegenwart eines Dialkylamins oder Alkalialkanolats zu einer Verbindung der Formel Ic 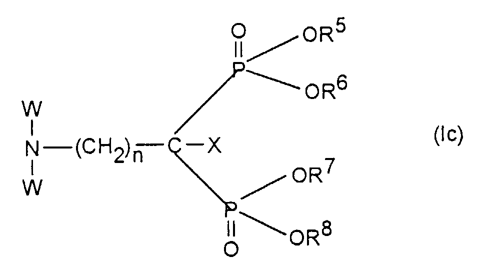 umsetzt, wobei R⁷ und R⁸ Wasserstoff oder (C₁-C₅)-Alkyl, geradkettig oder verzweigt, bedeutet und X für Wasserstoffatom oder Hydroxy steht,
anschließend von der Verbindung der Formel Ic
A3) die Aminoschutzgruppe abspaltet, und man die Verbindung der Formel Id erhält 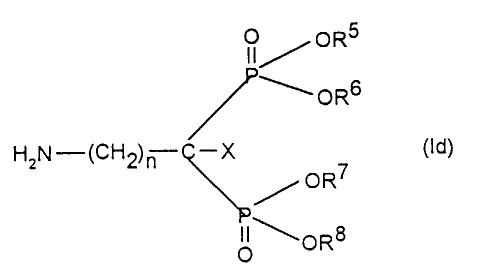
A4) anschließend die Verbindung der Formel Id mit einer tautomeren Verbindung der Formel IIIa oder IIIb 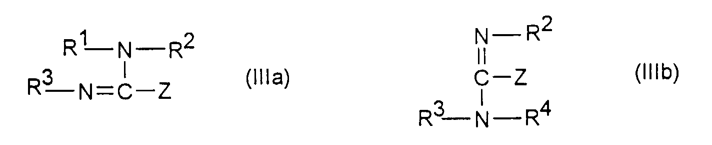 wobei Z ein Rest der nachstehenden Gruppe
1) Methylmercaptan, 2) Ethylmercaptan oder 3) 3,5-Dimethylpyrazol-1-carbonsäureamidinnitrat ist
und R¹, R², R³ oder R⁴ Wasserstoff, Benzimidazol oder O,O-Diisobutylphosphoryl bedeuten
in Gegenwart eines inerten Lösungsmittels zu den tautomeren Verbindungen des Anspruchs 1 umsetzt, oder
B) eine Verbindung der Formel IV 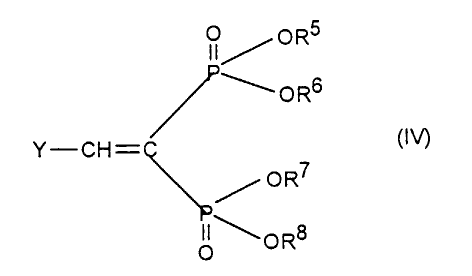 wobei R⁵, R⁶, R⁷, R⁸ die bei A1) bis A4) genannten Bedeutungen haben und Y Wasserstoff bedeutet,
B1) mit einer Verbindung der Formel V, wobei Z Amino bedeutet, in Gegenwart eines inerten Lösungsmittels zu einem Bisphosphonsäureester des Anspruchs 1 umsetzt, und gegebenenfalls
B2) den Bisphosphonsäureester des Anspruchs 1 in die entsprechende Bisphosphonsäure umsetzt, oder
C) eine Verbindung der Formel V 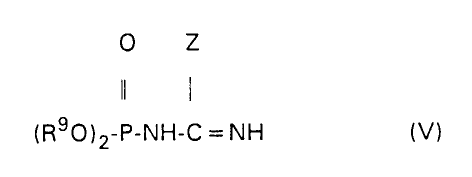 wobei Z die in A4) genannte Bedeutung hat und R⁹ (C₁-C₅)-Alkyl bedeutet,
C1) mit einer Verbindung der Formel Id zu einem Bisphosphonsäureester des Anspruchs 1 umsetzt und anschließend gegebenenfalls
C2) den Bisphosphonsäureester in die Bisphosphonsäure des Anspruchs 1 umsetzt, oder
D) eine Verbindung der Formel IV mit einer Verbindung der Formel IIIa oder Illb, wobei Z Amino bedeutet, und R¹, R², R³ und R⁴ die unter A4) genannten Bedeutungen haben, zu einer Verbindung des Anspruchs 1 umsetzt, oder
E) eine Verbindung der Formel Id
E1) mit einer Verbindung der Formel VI 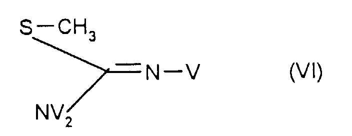 wobei V eine Aminoschutzgruppe ist umsetzt und man die Verbindung der Formel le erhält 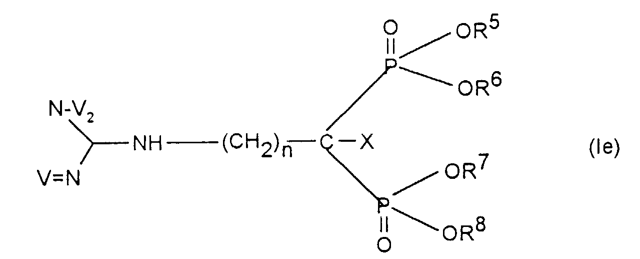
E2) anschließend die gegebenenfalls vorhandene Aminoschutzgruppe abgespaltet, und man eine tautomere Verbindung des Anspruchs 1 erhält.
F) die in B2, C2 oder E2 erhaltenen Bisphosphonsäuren des Anspruchs 1 durch Umsetzung mit einer physiologisch verträglichen Base in die entsprechenden Salze überführt.

3. Arzneimittel, enthaltend eine wirksame Menge von mindestens einer Verbindung des Anspruchs 1 und/oder 1-(Aminoiminomethyl)amino-methan-1,1-bisphosphonsäuretetraethylester und/oder dessen physiologisch verträglichen Salzes neben physiologisch annehmbaren Hilfs- und Trägerstoffen, gegebenenfalls weiteren Zusatzstoffen und/oder anderen Wirkstoffen.

4. Verfahren zur Herstellung eines Arzneimittels nach Anspruch 3, dadruch gekennzeichnet, daß man mindestens eine Verbindung gemäß Anspruch 3 und/oder mindestens ein physiologisch verträgliches Salz einer solchen Verbindung mit physiologisch annehmbaren Hilfs- und Trägerstoffen, gegebenenfalls weiteren Zusatzstoffen und/oder anderen Wirkstoffen in eine geeignete Darreichungform bringt.

5. Anwendung einer Verbindung gemäß Anspruch 1 oder des 1-(Aminoiminomethyl)amino-methan-1,1-bisphosphonsäuretetraethylesters zur Herstellung eines Arzneimittels zur Prophylaxe oder Behandlung von Osteoporose.

## Claims

1. Tetraethyl 2-(O,O-diisobutylphosphoryl)-(amino iminomethyl)aminoethane-1,1-bisphosphonate, 2-((aminoiminomethyl)amino)ethane-1,1-bisphosphonic acid, tetraethyl 2-((benzimidazolaminoiminomethyl)amino)ethane-1,1-bisphosphonate, 2-(benzimidazolaminoiminomethyl)aminoethane-1,1-bisphosphonic acid, 4-(aminoiminomethyl)aminobutane-1,1-bisphosphonic acid.

2. A process for the preparation of a compound as claimed in claim 1, wherein
A) a compound of the Formula Ia 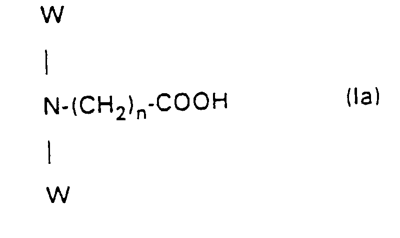 and/or the corresponding anhydride or acid chloride of the compound of the formula Ia, where W is an amino protective group and n is 2 or 4,
A1) is reacted with a compound of the formula IIa and/or IIb
P(OR⁵)₃ (IIa)
P(OR⁶)₃ (IIb)
where R⁵ and R⁶ independently of one another are (C₁-C₅)-alkyl, straight-chain or branched,
to give a compound of the formula Ib 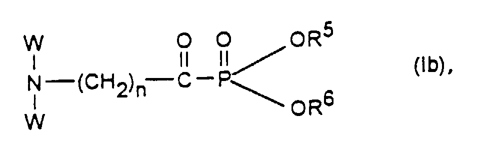 then the compound of the formula Ib
A2) is reacted with a compound of the formula IIc and/or IId
HP(O)(OR⁷)₂ (IIc)
P(OH)(OR⁸)₂ (IId)
in the presence of a dialkylamine or alkali metal alkoxide to give a compound of the formula Ic 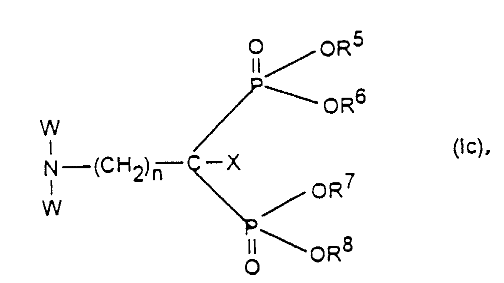 where R⁷ and R⁸ are hydrogen or (C₁-C₅)-alkyl, straight-chain or branched, and X is a hydrogen atom or hydroxyl,
then, from the compound of the formula Ic,
A3) the amino protective group is removed, and the compound of the formula Id is obtained 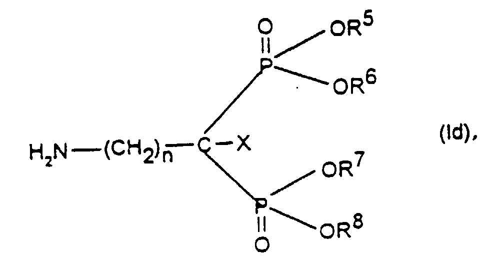
A4) then the compound of the formula Id is reacted with a tautomeric compound of the formula IIIa or IIIb 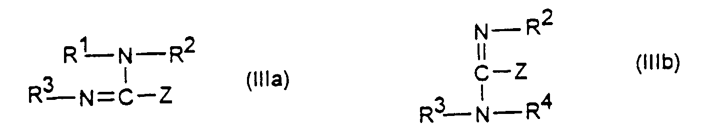 where Z is a radical of the group below
1) methylmercaptan, 2) ethylmercaptan or 3) 3,5-dimethylpyrazole-1-carboxamidine nitrate and R¹, R², R³ or R⁴ is hydrogen, benzimidazole or O,O-diisobutylphosphoryl
in the presence of an inert solvent to give the tautomeric compounds of claim 1, or
B) a compound of the formula IV 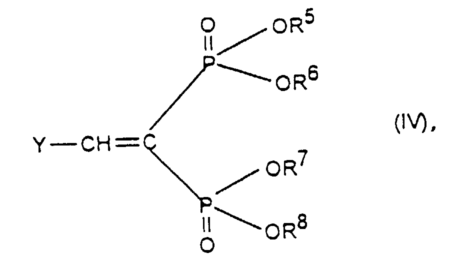 where R⁵, R⁶, R⁷, R⁸ have the meanings mentioned in A1) to A4) and Y is hydrogen,
B1) is reacted with a compound of the formula V, where Z is amino, in the presence of an inert solvent to give a bisphosphonic acid ester of claim 1, and, if appropriate,
B2) the bisphosphonic acid ester of claim 1 is converted into the corresponding bisphosphonic acid, or
C) a compound of the formula V 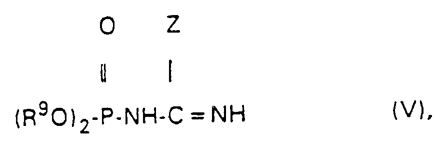 where Z has the meaning mentioned in A4) and R⁹ is (C₁-C₅)-alkyl,
C1) is reacted with a compound of the formula Id to give a bisphosphonic acid ester of claim 1 and then, if appropriate,
C2) the bisphosphonic acid ester is converted into the bisphosphonic acid of claim 1, or
D) a compound of the formula IV is reacted with a compound of the formula IIIa or IIIb, where Z is amino, and R¹, R², R³ and R⁴ have the meanings mentioned under A4), to give a compound of claim 1, or
E) a compound of the formula Id
E1) is reacted with a compound of the formula VI 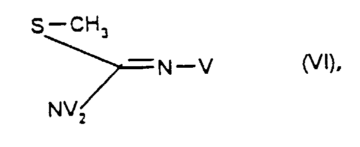 where V is an amino protective group and the compound of the formula Ie is obtained 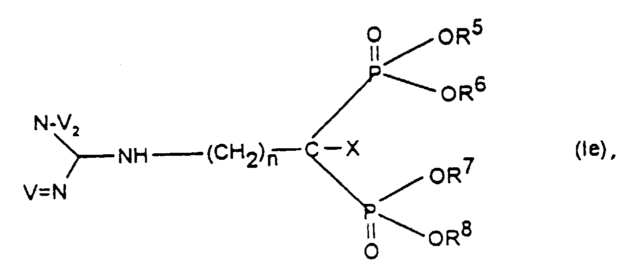
E2) then the amino protective group which is optionally present is removed, and a tautomeric compound of claim 1 is obtained,
F) the bisphosphonic acids of claim 1 obtained in B2, C2 or E2 are converted into the corresponding salts by reaction with a physiologically tolerable base.

3. A pharmaceutical comprising an efficacious amount of at least one compound of claim 1 and/or tetraethyl 1-(aminoiminomethyl)aminomethane-1,1-bisphosphonate and/or its physiologically tolerable salts, in addition to physiologically acceptable auxiliaries and excipients and, if appropriate, further additives and/or other active compounds.

4. A process for the production of a pharmaceutical as claimed in claim 3, -which comprises bringing at least one compound as set forth in claim 3 and/or at least one physiologically tolerable salt of such a compound into a suitable administration form with physiologically acceptable auxiliaries and excipients and, if appropriate, further additives and/or other active compounds.

5. The use of a compound as claimed in claim 1 or of tetraethyl 1-(aminoiminomethyl)aminomethane-1,1-bisphosphonate for the production of a pharmaceutical for the prophylaxis or treatment of osteoporosis.

## Revendications

1. 2-(O,O-diisobutylphosphoryl)-(amino-iminométhyl)-aminoéthane-1,1-bisphosphonate de tétraéthyle, acide 2-((amino-iminométhyl)amino)éthane-1,1-bisphosphonique, 2-((benzimidazolamino-iminométhyl)amine)éthane-1,1-bis-phosphonate de tétraéthyle, acide 2-(benzimidazolaminoiminométhyl)aminoéthane-1,1-bisphosphonique, acide 4-(aminoiminométhyl)aminobutane-1,1-bisphosphonique.

2. Procédé pour la préparation d'un composé selon la revendication 1, caractérisé en ce que
A) on fait réagir un composé de formule Ia 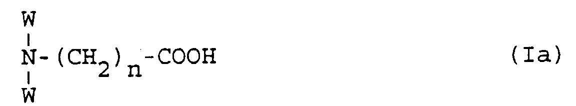 et/ou l'anhydride ou le chlorure d'acide correspondant du composé de formule IA,
W représentant un groupe amino protecteur de fonction amino et n étant égal à 2 ou 4,
A1) avec un composé de formule IIa et/ou de formule IIb
P(OR⁵)₃ (IIa)
P(OR⁶)₃ (IIb)
dans lesquelles R⁵ et R⁶ représentent, indépendamment l'un de l'autre, un groupe alkyle en C₁-C₅, à chaîne droite ou ramifié,
pour aboutir à un composé de formule Ib 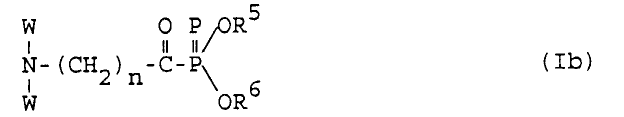 puis on fait réagir le composé de formule Ib
A2) avec un composé de formule IIc et/ou de formule IId
HP(O)(OR⁷)₂ (IIc)
P(OH)(OR⁸)₂ (IId)
en présence d'une dialkylamine ou d'un alcanolate de métal alcalin, pour aboutir à un composé de formule Ic 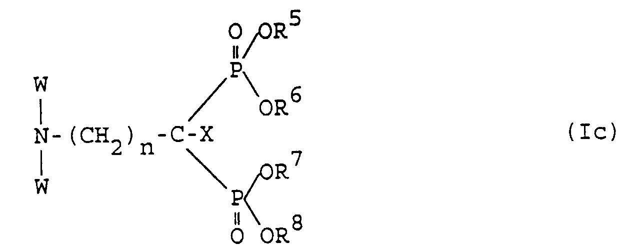 R⁷ et R⁸ représentant un atome d'hydrogène ou un groupe alkyle en C₁-C₅, à chaîne droite ou ramifié, et X représentant un atome d'hydrogène ou le groupe hydroxy,
et ensuite
A3) on élimine le groupe protecteur de la fonction amino du composé de formule Ic, pour obtenir le composé de formule Id 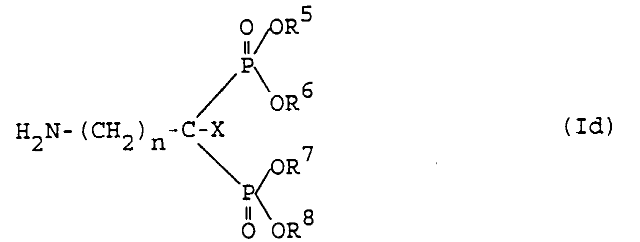
A4) et on fait ensuite réagir le composé de formule Id avec un composé tautomère de formule IIIa ou IIIb 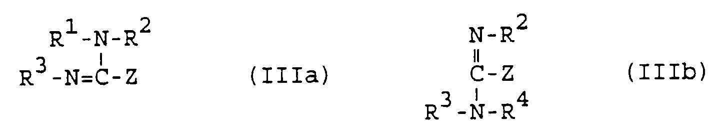 Z étant un radical choisi parmi les radicaux suivants:
1) méthylmercaptan, 2) éthylmercaptan ou 3) 3,5-diméthylpyrazole-1-carboxamidine-nitrate,
et R¹, R², R³ ou R⁴ représentant un atome d'hydrogène ou le groupe benzimidazole ou O,O-diisobutylphosphoryle,
en présence d'un solvant inerte, pour aboutir aux composés tautomères de la revendication 1, ou
B) on fait réagir un composé de formule IV 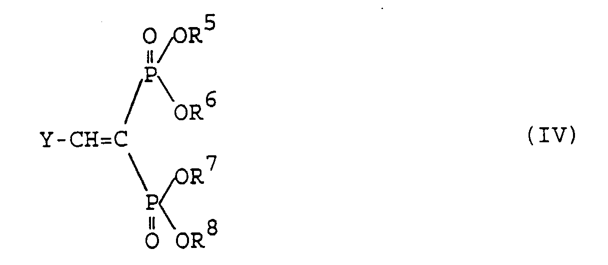 dans laquelle R⁵, R⁶, R⁷, R⁸ ont les significations données dans A1) à A4) et Y représente un atome d'hydrogène,
B1) avec un composé de formule V, dans lequel Z représente le groupe amino, en présence d'un solvant inerte, pour aboutir à un ester d'acide bisphosphonique de la revendication 1, et éventuellement
B2) on convertit l'ester d'acide bisphosphonique de la revendication 1 en l'acide bisphosphonique correspondant, ou
C) on fait réagir un composé de formule V 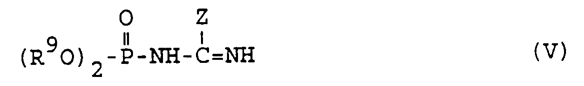 dans laquelle Z a la signification donnée en A4) et R⁹ représente un groupe alkyle en C₁-C₅,
C1) avec un composé de formule Id, pour aboutir à un ester d'acide bisphosphonique de la revendication 1, et ensuite, éventuellement
C2) on convertit l'ester d'acide bisphosphonique en l'acide bisphosphonique de la revendication 1, ou
D) on fait réagir un composé de formule IV avec un composé de formule IIIa ou IIIb, dans lequel Z représente le groupe amino, et R¹, R², R³ et R⁴ ont les significations données en A4), pour aboutir à un composé de la revendication 1, ou
E) on fait réagir un composé de formule Id
E1) avec un composé de formule VI 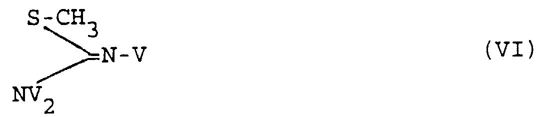 dans laquelle V est un groupe protecteur dela fonction amino, pour obtenir le composé de formule Ie 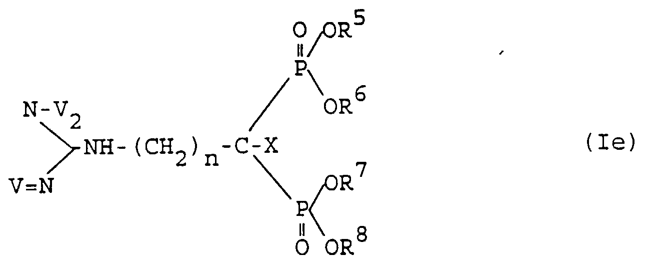
E2) on élimine ensuite le groupe protecteur de la fonction amino éventuellement présent, pour obtenir un composé tautomère de la revendication 1,
F) et on convertit les acides bisphosphoniques de la revendication 1, obtenus en B2, C2 ou E2, en les sels correspondants, par une réaction avec une base physiologiquement acceptable.

3. Médicament, contenant une quantité efficace d'au moins un composé de la revendication 1 et/ou de 1-(aminoiminométhyl)aminométhane-1,1-bisphosphonate de tétraéthyle et/ou d'un sel physiologiquement acceptable de celui-ci, en plus d'adjuvants et véhicules physiologiquement acceptables, éventuellement d'autres additifs et/ou d'autres substances actives.

4. Procédé pour la fabrication d'un médicament selon la revendication 3, caractérisé en ce que l'on met sous une forme d'administration approppriée au moins un composé tel que défini à la revendication 3 et/ou au moins un sel physiologiquement acceptable d'un tel composé, avec des adjuvants et véhicules physiologiquement acceptables, éventuellement d'autres additifs et/ou d'autres substances actives.

5. Utilisation d'un composé selon la revendication 1 ou du 1-(amino-iminométhyl)aminométhane-1,1-bisphosphonate de tétraéthyle, pour la fabrication d'un médicament destiné à la prophylaxie ou au traitement de l'ostéoporose.
